# EUROPÄISCHE PATENTSCHRIFT

(11) **EP 3 496 779 B1**
(45) Veröffentlichungstag und Bekanntmachung des Hinweises auf die Patenterteilung: **25.08.2021**
(21) Anmeldenummer: 17740608.9
(22) Anmeldetag: 06.07.2017
(51) Int. Cl.: A61M 1/16, A61M 1/36

(54) **OXYGENATOR MIT EINEM HEIZELEMENT**
OXYGENATOR COMPRISING A HEATING ELEMENT
OXYGÉNATEUR COMPRENANT UN ÉLÉMENT CHAUFFANT

(30) Priorität: 08.08.2016 DE 102016009534
(43) Veröffentlichungstag der Anmeldung: 19.06.2019
(73) Patentinhaber: Xenios AG, 74076 Heilbronn (DE)
(72) Erfinder: MATHEIS, Georg, 74076 Heilbronn (DE)
(74) Vertreter: Graf von Stosch, Andreas
(86) Internationale Anmeldenummer: PCT/DE2017/000147
(87) Internationale Veröffentlichungsnummer: WO 2018/028726

(56) Entgegenhaltungen:
- EP-A1- 2 848 269
- EP-A2- 0 621 047
- WO-A1-2013/012776
- WO-A2-2012/013925
- DE-A1-102011 052 188
- US-A- 3 929 414
- US-A1- 2012 277 654

## Beschreibung

Die Erfindung betrifft einen Oxygenator gemäß Anspruch 1 mit einer Gehäusewandung, die einen Gehäuseraum mit einem Bluteinlass, einem Blutauslass, einem Gaseinlass und einem Gasauslass begrenzt, und einem Heizelement, das im Oxygenator zwischen Bluteinlass und Blutauslass angeordnet ist, um durch den Gehäuseraum fließendes Blut zu temperieren.

Oxygenatoren sind medizinische Gasaustauscher, welche hauptsächlich in mehrtägigen Herz-Lunge-Therapien oder bei Operationen eingesetzt werden. Ein weiterer Anwendungszweck ist bspw. die Dialyse. Diese Oxygenatoren bieten neben dem Gasaustausch häufig auch die Möglichkeit, durch einen Gehäuseraum des Oxygenators fließendes Blut zu temperieren. In der Regel wird das Blut im Oxygenator erwärmt, da die Bluttemperatur im extrakorporalen Kreislauf, also außerhalb des Patientenkörpers, im Laufe der Zeit abnimmt und der Patient unterkühlt wird. Neben dieser Erwärmung gibt es auch die Möglichkeit, bei Operationen am Herzen die Temperatur des Blutes zu kühlen, um die Körpertemperatur zu senken.

Um die Blut- und Körpertemperatur eines Patienten während einer Operation oder bei längeren Therapieeinsätzen mit Gasaustauschern zu regulieren, werden Heater-Cooler (HC-Geräte) eingesetzt. Ein Heater-Cooler (HC-Gerät) ist ein externes Gerät, welches über Schläuche mit einem Oxygenator verbunden ist. Im HC-Gerät wird Wasser durch Metallstreben geleitet und erhitzt oder gekühlt. Das Wasser wird anschließend zum Oxygenator geleitet und fließt durch Wärmetauschermatten aus Hohlfasern oder spezielle meist metallische Kanäle im Oxygenator, an denen das Blut vorbeigeleitet wird. Ein derartiger Oxygenator ist in der EP 765 683 B1 beschrieben. Die US2012/0277654 A1 offenbart einen Oxygenator mit einem Heizelement. Die WO2012/013925 A2 offenbart ein Verfahren zum Regeln der Wärmeabgabe an einem Heizelement eines Oxygenators.

Derartige Oxygenatoren sind praktisch im Einsatz. Die verwendeten Heater-Cooler-Geräte arbeiten jedoch mit einem Wasserbad, das während der Nutzung verschmutzt und die Luft der Umgebung kontaminieren kann. Die HC-Geräte sind wegen des Wasserbades und der Kühleinrichtung sehr schwer und unmobil. Sie müssen regelmäßig gereinigt werden, da sie in der Nähe des Oxygenators und somit im Krankenhaus z.B. in Operationssälen oder Intensivstationen eingesetzt werden.

Der Erfindung liegt daher die Aufgabe zugrunde, einen Oxygenator weiterzuentwickeln.

Diese Aufgabe wird mit einem gattungsgemäßen Oxygenator gemäß Anspruch 1 gelöst, der einen elektrischen Anschluss aufweist und bei dem das Heizelement einen elektrischen Widerstand aufweist.

Der Erfindung liegt die Erkenntnis zugrunde, dass es nicht ausreicht, am Blut-oder Gaseinlass oder am Blut- oder Gasauslass den Blut- oder den Gasstrom zu erwärmen, und dass eine Erwärmung im Oxygenator zwischen Bluteinlass und Blutauslass mittels Flüssigkeit führenden Röhren ein aufwändiges Zusatzgerät benötigt. Dadurch, dass das Heizelement im Oxygenator einen elektrischen Widerstand aufweist, kann mit dem elektrischen Anschluss am Oxygenator durch eine angelegte Spannung der Widerstand des Heizelementes zur Erwärmung des Heizelementes genutzt werden. Da zur Erwärmung der elektrische Widerstand verwendet wird, kann auf ein HC-Gerät verzichtet werden und es wird nur eine Spannungsquelle benötigt.

Das Heizelement kann in der äußeren Wandung des Oxygenators angeordnet sein, um im Gehäuseraum fließendes Blut zu erwärmen. Besonders vorteilhaft ist es jedoch, wenn das Heizelement im Gehäuseraum angeordnet ist. Dies ermöglicht den Einsatz großer Heizflächen und dadurch einen geringen Temperaturunterschied zwischen Heizelement und Blut. Dadurch wird eine Schädigung des Blutes vermieden.

Je nach Einsatzzweck kann das Heizelement jedoch auch in der Gehäusewandung angeordnet sein. Dies ermöglicht einen einfachen Aufbau und insbesondere bei ebenen parallel gelegten Hohlfasermatten einen guten Wärmeübergang vom Heizelement auf das Blut.

Zwischen den Gasbereichen und den Blutbereichen des Oxygenators sind in der Regel semipermeable Materialien wie insbesondere Membranen angeordnet. Diese Membranen können ebene Folien oder Hohlfasern sein.

Zum Halten von planen oder rohrförmigen Membranen in einem Oxygenator werden Vergussmaterialien bspw. aus Kunststoff eingesetzt. Daher ist es vorteilhaft, wenn der Oxygenator eine Vergussschicht zum Halten von Fluidleitungen aufweist und das Heizelement in dieser Vergussschicht oder zumindest auch in dieser Vergussschicht angeordnet ist.

Bei der Erhitzung von Blut muss darauf geachtet werden, dass auch nur durch eine bereichsweise Überhitzung des Blutes keine Blutschädigungen auftreten. Daher wird vorgeschlagen, dass der Oxygenator mindestens einen Temperatursensor aufweist. Besonders vorteilhaft ist es, wenn im Oxygenator an verschiedenen Stellen Temperatursensoren vorgesehen werden, um sicherzustellen, dass in keinem Bereich zu hohe Temperaturen auftreten. Die Temperatursensoren sollten daher nach Möglichkeit zumindest auch in Bereichen angeordnet sein, in denen eine langsamere Blutflussgeschwindigkeit als in anderen Bereichen des Gehäuseraumes oder in denen eine langsamere Blutflussgeschwindigkeit als die mittlere Blutflussgeschwindigkeit im Gehäuseraum vorliegt und somit das Risiko einer Überhitzung bestehen kann.

Über die am Heizelement angelegte Spannung kann die Temperatur des Heizelementes variiert werden und es ist daher sinnvoll, dass der erfindungsgemäße Oxygenator eine Temperaturregeleinrichtung aufweist.

Vorteilhaft ist es, wenn die Temperatur an einer oder mehreren Stellen in bestimmten Zeitintervallen gemessen wird. Die Frequenz kann durch einen Algorithmus vorgegeben sein. Dadurch können risikohafte Überhitzungen vermieden werden. Hierbei spricht man von Pulsweitenmodulation.

In vielen Fällen steht der Oxygenator in Verbindung mit einer Konsole, über die beispielsweise die Durchströmung des Oxygenators mit Gas oder Blut gesteuert werden kann. Eine derartige Konsole ist eine Steuerelektronik zum Steuern oder Regeln des Oxygenatoreinsatzes. Über eine derartige Konsole kann somit auch das Heizelement angesteuert werden und diese Ansteuerung kann in Abhängigkeit von anderen an der Konsole vorliegenden Daten oder Verfahrensparametern geregelt werden, wie Blut oder Gasstrom und der Temperatur im Oxygenator.

Die Erfindung sieht vor, dass die Temperaturregelung die Temperatur des Heizelementes an verschiedenen Orten individuell einstellt oder regelt. Dies ermöglicht es, auf der Grundlage der üblichen Strömungsgeschwindigkeiten im Oxygenator an unterschiedlichen Stellen mit unterschiedlicher Intensität zu heizen.

Dafür wird vorgesehen, dass das Heizelement an unterschiedlichen Orten im Gehäuseraum eine unterschiedliche Heizleistung abgibt. Erfindungsgemäß wird die Heizleistung in Abhängigkeit von einem gemessenen Durchfluss des Blutes durch den Oxygenator oder von der Leistung einer den Durchfluss beeinflussenden Pumpe eingestellt.

Eine Ausführungsvariante sieht vor, dass das Heizelement mehrere an verschieden Orten des Oxygenators positionierbare Heizteilelemente aufweist. Diese Heizteilelemente können dann getrennt voneinander individuell angesteuert werden, um im Oxygenator eine bestimmte Heizintensitätsverteilung zu erzielen und diese gegebenenfalls auch während des Betriebs des Oxygenators zu ändern.

Es kann jedoch auch ein Heizelement vorgesehen werden, das mehrere getrennt voneinander ansteuerbare Heizteilelemente aufweist.

Ein Zusatzeffekt wird dadurch erzielt, dass das Heizelement im Oxygenator zwischen Gaseinlas und Gasauslass angeordnet ist, um auch durch den Gehäuseraum fließendes Gas zu temperieren. Dadurch kann vor allem auch Kondensatbildung vermieden werden.

Eine einfache Ausführungsform eines Oxygenators sieht einen Oxygenator mit einer Gehäusewandung vor, die nur vier nach außen führende Fluiddurchgänge aufweist. Davon können zwei Fluiddurchgänge für den Gaseinlass und Gasauslass und zwei Fluiddurchgänge für den Bluteinlass und Blutauslass verwendet werden.

Ein Konnektor bezeichnet eine Anschlussmöglichkeit von Schläuchen am Oxygenator. In einem derartigen Konnektor kann ein Teil des Heizelements angeordnet sein.

Vorteilhaft ist es, wenn der Oxygenator eine Wärmeleiteinrichtung für eine Wärmeleitung zum Heizelement aufweist. Wenn das Heizelement beispielsweise als erwärmbares Metallteil ausgebildet ist, kann dieses zur Vergrößerung der Oberfläche oder um einen Kontakt zwischen Blut- und Metallteil zu verhindern mit einer Wärmeleiteinrichtung umgeben sein. Diese Wärmeleiteinrichtung leitet dann die Wärme vom Heizelement an eine Oberfläche, die mit dem Blut in Verbindung steht und vorzugweise größer ist als die Oberfläche des Heizelementes. Eine derartige Oberfläche kann die Oberfläche eines Netzes oder einer Folie sein.

Die Wärmeleiteinrichtung sollte insbesondere dazu dienen, eine Wärmeverteilung vom Heizelement im Gehäuseraum zu ermöglichen.

Eine vorteilhafte Ausführungsvariante sieht vor, dass der Oxygenator eine Isolationsschicht oder eine Vakuumschicht aufweist, um im Gehäuseraum fließendes Blut zu isolieren. Eine Isolationsschicht und eine Reflexionsschicht kann auch so ausgebildet sein, dass sie geöffnet werden kann, um auch Wärme wieder leicht abzugeben, um den Oxygenator zu kühlen und somit eine Überhitzung zu vermeiden. Außerdem können die Schichten auch partiell angeordnet sein oder partiell anordenbar sein.

Um Wärmestrahlung von im Gehäuseraum fließendem Blut zum Blut zurück zu reflektieren und somit auch die Abgabe von Wärmestrahlung am Oxygenator zu minimieren, wird vorgeschlagen, dass der Oxygenator eine Reflektionsschicht aufweist. Eine derartige Reflektionsschicht kann beispielsweise eine Metallfolie sein oder eine polierte Oberfläche.

Damit die Blutströmung im Oxygenator beobachtet werden kann, ist es vorteilhaft, wenn die Isolations-, Vakuum- und/oder Reflektionsschicht durchsichtig oder zumindest partiell durchsichtig ist. Hierfür kann beispielsweise ein engmaschiges Netz, eine gelochte Folie oder eine Folie mit durchsichtigen Fensterbereichen vorgesehen sein.

Eine einfache Ausführungsmöglichkeit sieht vor, dass der Widerstand ein Metall, vorzugsweise Kupfer oder eine Kupfernickellegierung ist. Besonders vorteilhaft ist es, wenn der Widerstand ein Metall aufweist, dessen elektrischer Widerstand bei Erwärmung sinkt. Hierfür werden Kaltleiter oder PTC-Heizelemente vorgeschlagen, um eine materialgebundene Regulierung zu erzielen. Dies hilft, Überhitzungen des Blutes im Oxygenator zu vermeiden.

Weitere Ausführungsvarianten sehen vor, dass der Widerstand ein Kunststoff oder Kohlenstoff oder Grafit aufweist. Eine effektive einfache Wärmeverteilung im Oxygenator wird erreicht, wenn das Heizelement Heizdrähte aufweist.

Zwischen dem Heizdraht und dem Blut sollte ein Abstand von 1 bis 30 mm vorgesehen werden, um blutkritische Temperaturen zu vermeiden. Hierzu kann eine Isolier-oder Wärmeverteilschicht mit einer Dicke von mehr als 1 mm vorgesehen werden.

Die Heizdrähte können beispielsweise spiralförmig oder parallel zueinander angeordnet sein und sie sind besonders bevorzugt gleichverteilt im Gehäuseraum angeordnet.

Eine einfache Ausführungsvariante, die sich vor allem für zylinderförmige Oxygenatoren eignet, sieht vor, dass der Oxygenator eine zentrale Öffnung mit einem dornförmigen Halteelement aufweist. Dann kann das dornförmige Halteelement auch das Heizelement aufweisen, um im Gehäuseraum fließendes Blut zu erwärmen.

Verfahrensmäßig wird die der Erfindung zugrunde liegende Aufgabe durch ein Verfahren gemäß Anspruch 20 zum Regeln der Wärmeabgabe an einem Heizelement eines Oxygenators gelöst, bei dem der Durchfluss des Blutes durch den Oxygenator und die Leistung einer der Durchfluss beeinflussenden Pumpe gemessen werden und in Abhängigkeit davon die Heizleistung eingestellt wird. Dabei kann das Heizelement mehrere getrennt voneinander ansteuerbare Heizteilelemente aufweisen, die so angesteuert werden, dass die Temperaturdifferenz zwischen der Temperatur des Blutes am Heizteilelement und der Temperatur des Heizeilelementes einen vorbestimmten Wert nicht übersteigt. Diese Verfahren eignen sich besonders für einen erfindungsgemäßen Oxygenator.

Ausführungsbeispiele erfindungsgemäßer Oxygenatoren sind in der Zeichnung dargestellt und werden im Folgenden näher beschrieben.

Es zeigt
Figur 1 einen bekannten Oxygenator mit Blut-, Gas-, und Wasserströmung,
Figur 2 schematisch einen elektrisch beheizten Oxygenator,
Figur 3 eine Draufsicht auf den in Figur 2 gezeigten Oxygenator,
Figur 4 schematisch einen Oxygenator mit geschichteten Membranfasermatten und netzartigen Heizdrähten,
Figur 5 schematisch einen Oxygenator mit geschichteten Matten und parallel angeordneten Heizdrähten,
Figur 6 schematisch das Zusammenwirken von Komponenten für eine Algorithmus,
Figur 7 schematisch die mittleren Temperaturen über der Zeit,
Figur 8 schematisch die Temperatur über der Zeit an einem ersten Ort und
Figur 9 schematisch die Temperatur über der Zeit an einem zweiten Ort.

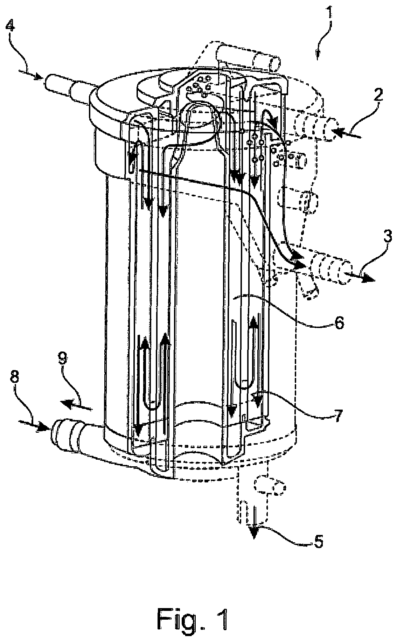

Der in Figur 1 gezeigte Oxygenator 1 hat einen Bluteinlass 2 und einen Blutauslass 3. Zur Gasversorgung sind ein Gaseinlass 4 und ein Gasauslass 5 vorgesehen. Im Wärmetauscher sind im radial inneren Bereich wasserdurchströmte Hohlfasern und im radial äußeren Bereich gasdurchströmte semipermeable Hohlfasern vorgesehen. Dadurch erfolgt radial innen eine Erwärmung durch am Wassereinlass 8 eintretendes und am Wasserauslass 9 austretendes Wasser, während im radial äußeren Bereich 7 ein Gasaustausch stattfindet. Zur genaueren Erläuterung eines derartigen Oxygenators wird auf die EP 765 683 B1 verwiesen.

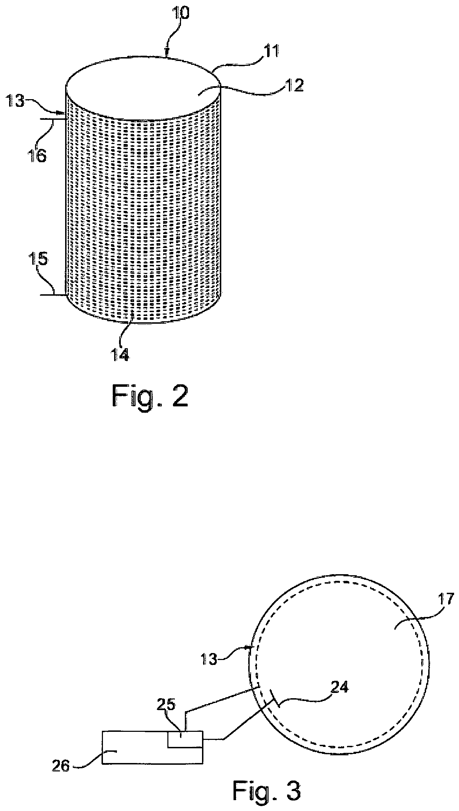

Bei dem in Figur 2 schematisch gezeigten Oxygenator bleibt der prinzipielle Aufbau im Wesentlichen erhalten und es wird auf den Wassereinlass 8, den Wasserauslass 9 und die wasserdurchströmten Hohlfasen verzichtet. Der Oxygenator 10 hat eine Gehäusewandung 11, die einen Gehäuseraum 12 umgibt. Dieser Gehäuseraum 12 hat, wie in Figur 1 gezeigt, einen Bluteinlass 2 und einen Blutauslass 3 und einen Gaseinlass 4 und einen Gasauslass 5. Als Heizelement 13 dient ein in der Gehäusewandung 11 angeordneter Heizdraht 14, der in gleichmäßigen Abständen um den Gehäuseraum 12 gewickelt ist. Der Heizdraht 14 bildet einen elektrischen Widerstand, der Wärme erzeugt, wenn am elektrischen Anschluss 15, 16 eine Spannung angelegt wird.

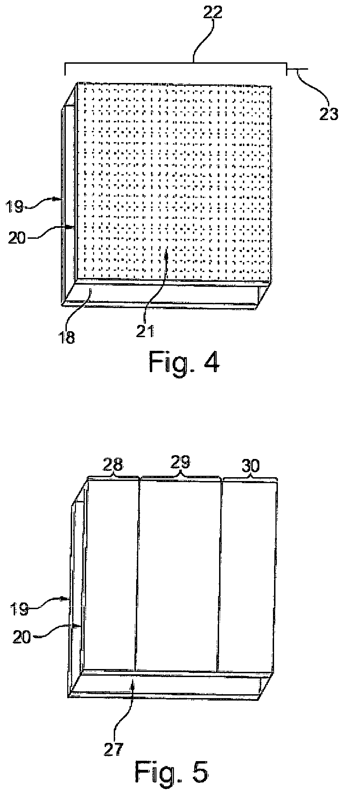

Der Oxygenator kann, wie in den Ausführungsbeispielen der Figuren 1 bis 3, aufgewickelte Hohlfasermatten 17 aufweisen, die im Gehäuseraum 12 angeordnet sind, oder, wie in den Figuren 4 und 5 gezeigt, gestapelte Matten 18 aus Hohlfasermembranen, die zwischen zwei Platten 19 und 20 angeordnet sind. Im Ausführungsbeispiel der Figur 4 ist in den Platten netzartig eine Drahtstruktur 21 eingearbeitet, die mit einem elektrischen Anschluss 22, 23 in Verbindung stehen. Bei der netzartigen Drahtstruktur liegt entweder nur eine Richtung des Gitters, das heißt von links nach rechts oder von oben nach unten am Strom an und die andere Richtung dient zur Wärmeleitung mit einem anderen nicht stromleitenden Material oder zwischen zwei stromleitenden Drähten der verschiedenen Richtungen ist eine Isolationsschicht vorgesehen, die einen Kurzschluss vermeidet. Die Figur 5 zeigt umwickelte Platten 19 und 20.

Die Figur 3 zeigt schematisch einen Temperatursensor 24, der mit einer Temperaturregeleinrichtung 25 in Verbindung steht, die in einer Konsole 26 aufgenommen ist.

Das in Figur 5 gezeigte Heizelement 27 besteht aus mehreren schematisch andeuteten Heizteilelementen 28, 29 und 30, die so angesteuert werden können, dass ein bestimmter Temperaturunterschied zwischen Heizteilelement und Bluttemperatur am Heizteilelement nicht überschritten wird.

Sämtliche Heizdrähte sind isoliert, um eine Überhitzung des Blutes und eine direkte Berührung zwischen Heizdraht und Blut zu vermeiden und um die Wärmeabgabe auf eine größere Oberfläche zu verteilen. Diese Isolierung kann auch strukturiert sein, um den Wärmeübergang zu verbessern.

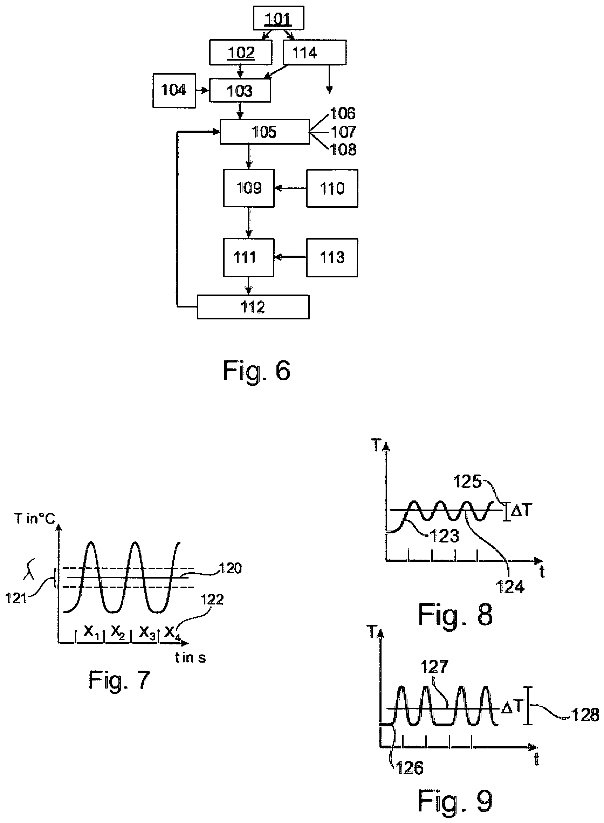

In den Figuren 6 bis 9 ist die algorithmische Steuerung nach Zeitintervallen in einer optimierten Form dargestellt. Aus einem realen Gasaustauscher wird ein Modell ermittelt. In diesem Gasaustauschermodell, das in der Figur 6 als Oxygenatormodell 114 zur Messpunktlokalisation eingezeichnet ist, werden Messpunkte definiert und es werden die Materialeigenschaften ermittelt, die als Parameter für die Berechnungen benötigt werden. An allen Messpunkten kann die Temperatur abgenommen werden.

In die Konsole werden durch die in einem ECMO-System bereits vorhandenen Sensoren, Blut- und Gasparameter eingespeist (KD). Anschließend wird eine Wunschtemperatur mit einer gemessenen Temperatur unter Berücksichtigung von Konsolenwerten (KD) verglichen. Dies wird individuell für jeden Messpunkt und jedes Heizelement vorgenommen. Dadurch ergeben sich verschiedene Toleranzen zwischen Messwert und Wunschtemperatur. Anschließend wird aus allen Parametern für jedes Heizelement die passende Heizfrequenz mit Heizhäufigkeit und Heizintensität gewählt, um möglichst blutschonend die Wunschtemperatur zu erreichen und anschließend zu halten. Diese Frequenzen können in einer Tabelle abgespeichert werden, um die Steuerung des Oxygenators später zu erleichtern.

In dem in Figur 6 dargestellten Algorithmus legt der Benutzer 101 die Wunschtemperatur 102 fest, die in die Konsole 103 eingegeben wird. Darüber hinaus werden die Blutfluss-, Gasfluss- und Druckparameter 104 in die Konsole eingegeben. Die Konsole veranlasst eine Temperaturmessung 105 um mittlere Temperaturen 106, 107 und 108 an unterschiedlichen Messpunkten des Oxygenators zu ermitteln. Der Vergleich 109 der Wunschtemperatur (WT) und der mittleren gemessenen Temperatur an unterschiedlichen Orten führt zum Differenzwert (AG). Dieser Wert wird mit den festgelegten Temperaturabweichungen 110 an unterschiedlichen Orten und den Konsolendaten (KD) wie beispielsweise dem Blutfluss verrechnet. Daraus ergibt sich die Grundlage für eine individuelle Regelung 111 der Heizelemente auf der Basis des Abgleiches (AG) der Temperaturabweichungen (AT) und den Konsolendaten (KD). Mit diesen Werten können die Heizelemente 112 angesteuert werden. Außerdem kann die individuelle Regelung noch vom Heizalgorithmus 113 beeinflusst werden, der aus einer Tabelle ausgewählt wird und sich aus den gemessenen Parametern ergibt.

Die Wärmeabgabe der Heizelemente 112 wirkt auf die mit der Temperaturmessung gemessenen Temperaturen 105, wodurch eine Rückkopplung auf die gemessenen Temperaturen entsteht.

Hierzu zeigt die Figur 7 die Wunschtemperatur 120 in einem Koordinatensystem mit der Temperatur in °C über der Zeit in Sekunden. Der Lamda-Wert deutet die Wärmeleitfähigkeit an, die durch die Materialkonstanten beeinflusst ist und die dazu führt, dass Temperaturspitzen abgefangen werden. Mit xl, x2, x3 und x4 sind beispielhaft Temperaturmesspunkte 122 bezeichnet.

In den Figuren 8 und 9 ist für zwei Messorte der Temperaturverlauf über der Zeit dargestellt. Dabei wird der Temperaturverlauf 123 an einem ersten Messort in Figur 8 über der Zeit als Wellenlinie dargestellt, die um eine Temperatur 124 schwankt und ein Delta T (Δ T) 125 definiert. In entsprechender Weise ist in Figur 9 an einem zweiten Ort die aktuelle Temperatur 126 über einer mittleren Temperatur 127 aufgezeichnet, wodurch sich eine Temperaturabweichung Delta T (Δ T) 128 ergibt.

## Patentansprüche

1. Oxygenator (10) mit einer Gehäusewandung (11), die einen Gehäuseraum (12) mit einem Bluteinlass (2), einem Blutauslass (3), einem Gaseinlass (4) und einem Gasauslass (5) begrenzt, einem Heizelement (13), das einen elektrischen Widerstand (14) aufweist und im Oxygenator zwischen Bluteinlass (2) und Blutauslass (3) angeordnet ist, um durch den Gehäuseraum (12) fließendes Blut zu temperieren, einem elektrischen Anschluss (15, 16) und einer Temperaturregeleinrichtung (25), ***dadurch gekennzeichnet, dass*** das Heizelement (13) an unterschiedlichen Orten im Gehäuseraum eine unterschiedliche Heizleistung abgibt.

2. Oxygenator nach Anspruch 1, ***dadurch gekennzeichnet, dass*** das Heizelement (13) im Gehäuseraum (12) angeordnet ist.

3. Oxygenator nach einem der vorhergehenden Ansprüche, ***dadurch gekennzeich-net, dass*** das Heizelement (13) in der Gehäusewandung (11) angeordnet ist.

4. Oxygenator nach einem der vorhergehenden Ansprüche, ***dadurch gekennzeich-net, dass*** er Membranen wie insbesondere Hohlfasern aufweist.

5. Oxygenator nach einem der vorhergehenden Ansprüche, ***dadurch gekennzeich-net, dass*** er eine Vergussschicht zum Halten von Fluidleitungen aufweist und das Heizelement (13) in dieser Vergussschicht angeordnet ist.

6. Oxygenator nach einem der vorhergehenden Ansprüche, ***dadurch gekennzeich-net, dass*** er mindestens einen Temperatursensor (24) aufweist.

7. Oxygenator nach einem der vorhergehenden Ansprüche, ***dadurch gekennzeich-net, dass*** die Temperaturregelung (25) die Temperatur des Heizelementes (13) an verschiedenen Orten individuell einstellt oder regelt.

8. Oxygenator nach einem der vorhergehenden Ansprüche, ***dadurch gekennzeich-net, dass*** das Heizelement (13) mehrere an verschiedenen Orten des Oxygenators positionierbare Heizteilelemente (28, 29, 30) aufweist.

9. Oxygenator nach Anspruch 8, **dadurch gekennzeichnet, dass** das Heizelement (27) mehrere getrennt voneinander ansteuerbare Heizteilelemente (28, 29, 30) aufweist.

10. Oxygenator nach einem der vorhergehenden Ansprüche, ***dadurch gekennzeich-net, dass*** das Heizelement (13) im Oxygenator zwischen Gaseinlass (4) und Gasauslass (5) angeordnet ist, um auch durch den Gehäuseraum (12) fließendes Gas zu temperieren.

11. Oxygenator nach einem der vorhergehenden Ansprüche, ***dadurch gekennzeich-net, dass*** er eine Wärmeleiteinrichtung für eine Wärmeleitung zum Heizelement (13) aufweist.

12. Oxygenator nach einem der vorhergehenden Ansprüche, ***dadurch gekennzeich-net, dass*** er eine Wärmeleiteinrichtung für eine Wärmeverteilung vom Heizelement (13) im Gehäuseraum (12) aufweist.

13. Oxygenator nach einem der vorhergehenden Ansprüche, ***dadurch gekennzeich-net, dass*** er eine Isolationsschicht aufweist, um im Gehäuseraum fließendes Blut zu isolieren.

14. Oxygenator nach einem der vorhergehenden Ansprüche, ***dadurch gekennzeich-net, dass*** er eine Reflexionsschicht aufweist, um Wärmestrahlung von im Gehäuseraum fließenden Blut zu reflektieren.

15. Oxygenator nach Anspruch 13 oder 14, ***dadurch gekennzeichnet, dass*** die Schicht durchsichtig oder partiell durchsichtig ist.

16. Oxygenator nach einem der vorhergehenden Ansprüche, ***dadurch gekennzeichnet, dass*** der Widerstand (14) ein Metall, vorzugsweise Kupfer oder eine Kupfernickellegierung ist.

17. Oxygenator nach einem der Ansprüche 1 bis 16, ***dadurch gekennzeichnet, dass*** der Widerstand (14) einen Kunststoff oder Kohlenstoff oder Graphit aufweist.

18. Oxygenator nach einem der vorhergehenden Ansprüche, ***dadurch gekennzeich-net, dass*** er eine zentrale Öffnung mit einem dornförmigen Halteelement aufweist.

19. Oxygenator nach Anspruch 18, ***dadurch gekennzeichnet, dass*** das dornförmige Halteelement das Heizelement aufweist.

20. Verfahren zum Regeln der Wärmeabgabe an einem Heizelement (13) eines Oxygenators (10), bei dem der Durchfluss des Blutes durch den Oxygenator (10) oder die Leistung einer den Durchfluss beeinflussenden Pumpe gemessen werden und in Abhängigkeit davon die Heizleistung eingestellt wird.

21. Verfahren nach Anspruch 20, ***dadurch gekennzeichnet, dass*** das Heizelement (13) mehrere getrennt voneinander ansteuerbare Heizteilelemente (28, 29, 30) aufweist, die so angesteuert werden, dass die Temperaturdifferenz zwischen der Temperatur des Blutes am Heizteilelement (28, 29, 30) und der Temperatur des Heizteilelements (28, 29, 30) einen vorbestimmten Wert nicht übersteigt.

## Claims

1. Oxygenator (10) with a housing wall (11), defining a housing chamber (12) with a blood inlet (2), a blood outlet (3), a gas inlet (4) and a gas outlet (5), a heating element (13) which has an electric resistor (14) and is arranged in the oxygenator between the blood inlet (2) and blood outlet (3) in order to control the temperature of the blood flowing through the housing chamber (12), an electric connection (15, 16) and a temperature control device (25), ***characterised in that** the* heating element (13) delivers a different heating power at different locations in the housing chamber (12).

2. The oxygenator according to claim 1, *characterised in thatthe* heating element (13) is arranged in the housing chamber (12).

3. The oxygenator according to any one of the preceding claims, *characterised in thatthe* heating element (13) is arranged in the housing wall (11).

4. The oxygenator according to any one of the preceding claims, ***characterised in that*** it has membranes, such as hollow fibres in particular.

5. The oxygenator according to any one of the preceding claims, ***characterised in that*** it has an encapsulation layer for holding fluid lines, and the heating element (13) is arranged in this encapsulation layer.

6. The oxygenator according to any one of the preceding claims, ***characterised in that*** it has at least one temperature sensor (24).

7. The oxygenator according to any one of the preceding claims, *characterised in thatthe* temperature controller (25) sets or controls the temperature of the heating element (13) individually at various locations.

8. The oxygenator according to any one of the preceding claims, *characterised in thatthe* heating element (13) has a plurality of heating sub-elements (28, 29, 30) positionable at various locations of the oxygenator.

9. The oxygenator according to claim 8, ***characterised in that*** the heating element (27) has a plurality of heating sub-elements (28, 29, 30) actuatable separately from one another.

10. The oxygenator according to any one of the preceding claims, ***characterised in that*** the heating element (13) is arranged in the oxygenator between gas inlet (4) and gas outlet (5) in order to also control the temperature of gas flowing through the housing chamber (12).

11. The oxygenator according to any one of the preceding claims, ***characterised in that*** it has a heat-conducting arrangement for conducting heat to the heating element (13).

12. The oxygenator according to any one of the preceding claims, *characterised in thatit* has a heat-conducting arrangement for distributing heat from the heating element (13) in the housing chamber (12).

13. The oxygenator according to any one of the preceding claims, ***characterised in that*** it has an insulation layer in order to insulate blood flowing in the housing chamber.

14. The oxygenator according to any one of the preceding claims, ***characterised in that*** it has a reflection layer in order to reflect heat radiation from blood flowing in the housing chamber.

15. The oxygenator according to claim 13 or 14, ***characterised in that*** the layer is transparent or partially transparent.

16. The oxygenator according to any one of the preceding claims, *characterised in thatthe* resistor (14) is a metal, preferably copper or a copper-nickel alloy.

17. The oxygenator according to any one of claims 1 to 16, *characterised in thatthe* resistor (14) comprises a plastic or carbon or graphite.

18. The oxygenator according to any one of the preceding claims, ***characterised in that*** it has a central opening with a mandrel-shaped holding element.

19. The oxygenator according to claim 18, ***characterised in that*** the mandrel-shaped holding element comprises the heating element.

20. A method for controlling the heat emission at a heating element (13) of an oxygenator (10), in which the flow rate of blood through the oxygenator (10) or the power of a pump influencing the flow rate are measured and the heating power is adjusted on this basis.

21. The method according to claim 20, ***characterised in that*** the heating element (13) has a plurality of heating sub-elements (28, 29, 30) actuatable separately from one another, which are actuated such that the temperature difference between the temperature of the blood at the heating sub-element (28, 29, 30) and the temperature of the heating sub-element (28, 29, 30) does not exceed a predetermined value.

## Revendications

1. Oxygénateur (10) avec une paroi de boîtier (11), qui délimite un espace de boîtier (12) avec une entrée de sang (2), une sortie de sang (3), une entrée de gaz (4) et une sortie de gaz (5), un élément chauffant (13), qui comprend une résistance électrique (14) et qui est disposé dans l'oxygénateur entre l'entrée de sang (2) et la sortie de sang (3), afin de réguler la température du sang s'écoulant à travers l'espace du boîtier (12), un raccordement électrique (15, 16) et un dispositif de régulation de la température (25), **caractérisé en ce que** l'élément chauffant (13) distribue une puissance de chauffage différente à différents endroits de l'espace du boîtier.

2. Oxygénateur selon la revendication 1, **caractérisé en ce que** l'élément chauffant (13) est disposé dans l'espace du boîtier (12).

3. Oxygénateur selon l'une des revendications précédentes, **caractérisé en ce que** l'élément chauffant (13) est disposé dans la paroi du boîtier (11).

4. Oxygénateur selon l'une des revendications précédentes, **caractérisé en ce qu'**il comprend des membranes tel que, plus particulièrement, des fibres creuses.

5. Oxygénateur selon l'une des revendications précédentes, **caractérisé en ce qu'**il comprend une couche de scellement pour le maintien de conduites de fluides et l'élément chauffant (13) est disposé dans cette couche de scellement.

6. Oxygénateur selon l'une des revendications précédentes, **caractérisé en ce qu'**il comprend au moins un capteur de température (24).

7. Oxygénateur selon l'une des revendications précédentes, **caractérisé en ce que** la régulation de température (25) règle ou régule la température de l'élément chauffant (13) individuellement à différents endroits.

8. Oxygénateur selon l'une des revendications précédentes, **caractérisé en ce que** l'élément chauffant (13) comprend plusieurs éléments chauffants partiels (28, 29, 30) pouvant être positionnés à différents endroits de l'oxygénateur.

9. Oxygénateur selon la revendication 8, **caractérisé en ce que** l'élément chauffant (27) comprend plusieurs éléments chauffants partiels (28, 29, 30) pouvant être contrôlés séparément les uns des autres.

10. Oxygénateur selon l'une des revendications précédentes, **caractérisé en ce que** l'élément chauffant (13) est disposé dans l'oxygénateur entre l'entrée de gaz (4) et la sortie de gaz (5), afin de réguler également la température du gaz s'écoulant à travers l'espace du boîtier (12).

11. Oxygénateur selon l'une des revendications précédentes, **caractérisé en ce qu'**il comprend un dispositif de conduction thermique pour une conduction thermique vers l'élément chauffant (13).

12. Oxygénateur selon l'une des revendications précédentes, **caractérisé en ce qu'**il comprend un dispositif de conduction thermique pour une conduction thermique de l'élément chauffant (13) vers l'espace du boîtier (12).

13. Oxygénateur selon l'une des revendications précédentes, **caractérisé en ce qu'**il comprend une couche d'isolation afin d'isoler le sang s'écoulant dans l'espace du boîtier.

14. Oxygénateur selon l'une des revendications précédentes, **caractérisé en ce qu'**il comprend une couche de réflexion afin de réfléchir le rayonnement thermique du sang s'écoulant dans l'espace du boîtier.

15. Oxygénateur selon la revendication 13 ou 14, **caractérisé en ce que** la couche est transparente ou partiellement transparente.

16. Oxygénateur selon l'une des revendications précédentes, **caractérisé en ce que** la résistance (14) est un métal, de préférence du cuivre ou un alliage de cuivre et de nickel.

17. Oxygénateur selon l'une des revendications 1 à 16, **caractérisé en ce que** la résistance (14) comprend une matière plastique ou du carbone ou du graphite.

18. Oxygénateur selon l'une des revendications précédentes, **caractérisé en ce qu'**il comprend une ouverture centrale avec un élément de maintien en forme de broche.

19. Oxygénateur selon la revendication 18, **caractérisé en ce que** l'élément de maintien en forme de broche comprend l'élément chauffant.

20. Procédé de régulation du dégagement de chaleur au niveau d'un élément chauffant (13) d'un oxygénateur (10), dans lequel le débit du sang à travers l'oxygénateur (10) ou la puissance d'une pompe contrôlant le débit est mesuré et la puissance de chauffage est ajustée en fonction de cette mesure.

21. Procédé selon la revendication 20, **caractérisé en ce que** l'élément chauffant (13) comprend plusieurs éléments chauffants partiels (28, 29, 30) pouvant être contrôlés séparément les uns des autres, qui sont contrôlés de façon à ce que la différence de température entre la température du sang au niveau de l'élément chauffant partiel (28, 29, 30) et la température de l'élément chauffant partiel (28, 29, 30) ne dépasse pas une valeur prédéterminée.
